# EUROPEAN PATENT APPLICATION

(11) **EP 4 227 682 A1**
(43) Date of publication of application: **16.08.2023**
(21) Application number: 21877414.9
(22) Date of filing: 27.09.2021
(51) Int. Cl.: G01N 33/53, G01N 33/543

(54) **HUMORAL ANTIBODY BIOMARKER FOR DETECTING ARTERY LESIONS, AND DETECTING CEREBROVASCULAR AND CARDIOVASCULAR DISORDER, DIABETES, CHRONIC NEPHROPATHY, OR SOLID CANCER**

(30) Priority: 08.10.2020 JP 2020170489
(71) Applicant: Fujikura Kasei Co., Ltd., Tokyo 174-0046 (JP); National University Corporation Chiba University, Chiba-shi, Chiba 263-8522 (JP)
(72) Inventor: KURODA, Hideyuki, Kuki-shi, Saitama 340-0203 (JP); NAKAMURA, Rika, Kuki-shi, Saitama 340-0203 (JP); TOMIYOSHI, Go, Kuki-shi, Saitama 340-0203 (JP); HIWASA, Takaki, Chiba-shi, Chiba 260-8670 (JP)
(74) Representative: Krauss, Jan
(86) International application number: PCT/JP2021/035474
(87) International publication number: WO 2022/075113

(57) **Abstract**

An object of the invention is to provide a comprehensive biomarker for artery lesions, and diabetes mellitus, heart disease, cerebrovascular disorder, cancer, kidney disease, etc., whose onset and progress can be suppressed by health care such as early diagnosis. The inventors have found that the object can be attained by determining the level of an antibody to protein KIAA0513 or a portion of the protein, the antibody being potentially present in a body fluid sample collected from a living body; and acquiring data showing a rise in the antibody level as data showing the presence of an artery lesion or the potential or actual presence of cerebrovascular or cardiovascular disorder, diabetes mellitus, chronic kidney disease, or a solid cancer.

## Description

### Technical Field

The present invention relates to means for acquiring data concerning a disease or disorder (hereinafter also referred to simply as "disease") or for detecting a disease. More particularly, the invention relates to means for acquiring data concerning an artery lesion or for detecting an artery lesion, and to means for acquiring data concerning cerebrovascular or cardiovascular disorder, diabetes mellitus, chronic kidney disease, solid cancer, etc. or detecting such diseases, these means being based on measuring the level of a biomarker which is a humoral antibody with respect to protein KIAA0513 or a peptide having a partial sequence of the protein.

### Background Art

A variety of disease-specific biomarkers have been found in recent years, and these biomarkers are further refined from many aspects including assessment of the disease condition, prediction of the onset and prognosis of the disease, and determination of a prophylactic/therapeutic target, through a simple blood test or the like. Conventional biomarkers include an enzyme marker and an antigen marker, and a DNA/RNA marker is reported in recent years. In contrast, a limited number of humoral antibody markers have been reported or implemented. Meanwhile, many antibody markers have been identified with respect to cerebrovascular and cardiovascular disorders. For example, Hsp60 (Non-Patent Document 1), RPA2 (Non-Patent Document 2), SOSTDC1 (Non-Patent Document 3), PDCD11 (Non-Patent Document 4), MMP1, CBX1, and CBX5 (Non-Patent Document 5) are reported as antibody markers with respect to acute ischemic stroke (AIS); ATP2B4 (Non-Patent Document 6), BMP-1 (Non-Patent Document 2, 6), DHPS (Non-Patent Document 7), SH3BP5 (Non-Patent Document 8), and prolylcarboxypeptidase (Non-Patent Document 9) are reported as markers with respect to atherosclerosis; nardilysin (NRD1) is reported as a marker with respect to acute coronary syndrome (Non-Patent Document 10); and TUBB2C (Non-Patent Document 11), insulin (Non-Patent Document 12), glutamic acid decarboxylase (Non-Patent Document 13), adiponectin (Non-Patent Document 14), and GADD34 (Non-Patent Document 15) are reported as markers with respect to diabetes mellitus (DM).

Regarding antibody biomarkers with respect to cancers, an anti-p53 antibody has been implemented as a marker which can be effectively employed for the diagnosis, monitoring, and prediction of prognosis of esophageal squamous cell carcinoma (ESCC) or head and neck cancer (Non-Patent Documents 16 and 17). The group to which the present inventors belong previously conducted screening through serological identification of antigens by recombinant cDNA expression cloning (SEREX), and found and reported Trop2/TACSTD2 (Non-Patent Document 18), SLC2A1 (Non-Patent Document 19), TRIM21 (Non-Patent Document 20), myomegalin (Non-Patent Document 21), makorin 1 (Non-Patent Document 22), ECSA (Non-Patent Document 23), and cyclin L2 (CCNL2) (Non-Patent Document 24) as antibody markers with respect to ESCC; FIR/PUF60 (Non-Patent Document 25) as an antibody marker with respect to colorectal cancer; and SH3GL1 (Non-Patent Document 26) and anti-filamin C (Non-Patent Document 27) as antibody markers with respect to glioma.

### Prior Art Documents

### Non-Patent Documents

Non-Patent Document 1: Kramer J, Harcos P, Prohaszka Z, et al. Frequencies of certain complement protein alleles and serum levels of anti-heat-shock protein antibodies in cerebrovascular diseases. Stroke. 2000; 31: 2648-2652. PMID: 11062289
Non-Patent Document 2: Machida T, Kubota M, Kobayashi E, et al. Identification of stroke-associated-antigens via screening of recombinant proteins from the human expression cDNA library (SEREX). J Translat Med. 2015; 13:71. PMID: 25890248
Non-Patent Document 3: Goto K, Sugiyama T, Matsumura R, et al. Identification of cerebral infarction-specific antibody markers from autoantibodies detected in patients with systemic lupus erythematosus. J Mol Biomark Diagnos. 2015; 6:2. doi.org/10.4172/2155-9929.1000219
Non-Patent Document 4: Yoshida Y, Wang H, Hiwasa T, et al. Elevation of autoantibody level against PDCD11 in patients with transient ischemic attack. Oncotarget. 2018; 9, 8836-8848. PMID: 29507658
Non-Patent Document 5: Wang H, Zhang XM, Tomiyoshi G, et al. Association of serum levels of antibodies against MMP1, CBX1, and CBX5 with transient ischemic attack and cerebral infarction. Oncotarget. 2018; 9: 5600-5613. PMID: 29464021
Non-Patent Document 6: Hiwasa T, Machida T, Zhang XM, et al. Elevated levels of autoantibodies against ATP2B4 and BMP-1 in sera of patients with atherosclerosis-related diseases. Immunome Res. 2015; 11:097. doi.org/10.4172/1745-7580.1000097
Non-Patent Document 7: Nakamura R, Tomiyoshi G, Shinmen N, et al. An anti-deoxyhypusine synthase antibody as a marker of atherosclerosis-related cerebral infarction, myocardial infarction, diabetes mellitus, and chronic kidney disease. SM Atheroscler J. 2017; 1:1001. doi: http://smjournals.com/atherosclerosis/in-press.php#x
Non-Patent Document 8: Hiwasa T, Tomiyoshi G, Nakamura R, et al. Serum SH3BP5-specific antibody level is a biomarker of atherosclerosis. Immunome Res. 2017; 13:132. doi: 10.4172/17457580.1000132
Non-Patent Document 9: Zhang XM, Wang H, Mine S, et al. Association of serum anti-prolylcarboxypeptidase antibody marker with atherosclerotic diseases accompanied by hypertension. J Mol Biomark Diagn. 2017; 8:361. doi: 10.4172/2155-9929.1000361
Non-Patent Document 10: Chen PM, Ohno M, Hiwasa T, et al. Nardilysin is a promising biomarker for the early diagnosis of acute coronary syndrome. Int J Cardiol. 2017; 243: 1-8. PMID: 28747015
Non-Patent Document 11: Hiwasa T, Zhang XM, Kimura R, et al. Association of serum antibody levels against TUBB2C with diabetes and cerebral infarction. Integ Biomed Sci. 2015; 1: 49-63. doi: 10.18314/gjbs.vli2.27
Non-Patent Document 12: Palmer JP, Asplin CM, Clemons P, et al. Insulin antibodies in insulin-dependent diabetics before insulin treatment. Science. 1983; 222:1337-1339. PMID: 6362005
Non-Patent Document 13: Baekkeskov S, Aanstoot H, Christgau S, et al. Identification of the 64K autoantigen in insulin dependent diabetes as the GABA-synthesizing enzyme glutamic acid decarboxylase. Nature. 1990; 347: 151-156. PMID: 1697648
Non-Patent Document 14: Hiwasa T, Zhang XM, Kimura R, et al. Elevated adiponectin antibody levels in sera of patients with atherosclerosis-related coronary artery disease, cerebral infarction, and diabetes mellitus. J Circ Biomark. 2016; 5:8. doi: 10.5772/63218
Non-Patent Document 15: Sugimoto K, Tomiyoshi G, Mori M, et al. Identification of serum anti-GADD34 antibody as a common marker of diabetes mellitus and Parkinson disease. J Alzheimers Dis Parkinsonism. 2017;7:358. doi: 10.4172/2161-0460.1000358
Non-Patent Document 16: Shimada H, Takeda A, Arima M, et al. Serum p53 antibody is a useful tumor marker in superficial esophageal squamous cell carcinoma. Cancer. 2000; 89: 1677-1683. PMID: 11042560
Non-Patent Document 17: Shimada H, Ochiai T, Nomura F, et al. Japan p53 Antibody Research Group. Titration of serum p53 antibodies in 1,085 patients with various types of malignant tumors: a multiinstitutional analysis by the Japan p53 Antibody Research Group. Cancer. 2003; 97: 682-689. PMID: 12548611
Non-Patent Document 18: Nakashima K, Shimada H, Ochiai T, et al. Serological identification of TROP2 by recombinant cDNA expression cloning using sera of patients with esophageal squamous cell carcinoma. Int J Cancer. 2004; 112: 1029-1035. PMID: 15386348
Non-Patent Document 19: Kuboshima M, Shimada H, Liu TL, et al. Identification of a novel SEREX antigen, SLC2A1/GLUT1, in esophageal squamous cell carcinoma. Int J Oncol. 2006; 28: 463-468. PMID: 16391802
Non-Patent Document 20: Kuboshima M, Shimada H, Liu TL, et al. Presence of serum tripartite motif-containing 21 antibodies in patients with esophageal squamous cell carcinoma. Cancer Sci. 2006; 97: 380-386. PMID: 16630135
Non-Patent Document 21: Shimada H, Kuboshima M, Shiratori T, et al. Serum anti-myomegalin antibodies in patients with esophageal squamous cell carcinoma. Int J Oncol. 2007; 30: 97-103. PMID: 17143517
Non-Patent Document 22: Shimada H, Shiratori T, Yasuraoka M, et al. Identification of makorin 1 as a novel SEREX antigen of esophageal squamous cell carcinoma. BMC Cancer. 2009; 9:232. PMID: 19604354
Non-Patent Document 23: Kagaya A, Shimada H, Shiratori T, et al. Identification of a novel SEREX antigen family, ECSA, in esophageal squamous cell carcinoma. Proteome Sci. 2011; 9:31. PMID: 21696638
Non-Patent Document 24: Shimada H, Ito M, Kagaya A, et al. Elevated serum antibody levels against cyclin L2 in patients with esophageal squamous cell carcinoma. J Cancer Sci Ther. 2015; 7: 60-66. doi:10.4172/1948-5956.1000326
Non-Patent Document 25: Kobayashi S, Hoshino T, Hiwasa T, et al. Anti-FIRs (PUF60) auto-antibodies are detected in the sera of early-stage colon cancer patients. Oncotarget. 2016; 7:82493-82503. PMID: 27756887
Non-Patent Document 26: Matsutani T, Hiwasa T, Takiguchi M, et al. Autologous antibody to src-homology 3-domain GRB2-like 1 specifically increases in the sera of patients with low-grade gliomas. Exp Clin Cancer Res. 2012; 31:85. PMID: 23050879
Non-Patent Document 27: Adachi-Hayama M, Adachi A, Shinozaki N, et al. Circulating anti-filamin C antibody as a potential serum biomarker for low-grade gliomas. BMC Cancer. 2014; 14:452. PMID: 24946857
Non-Patent Document 28: Makrilia N, Lappa T, Xyla V, et al. The role of angiogenesis in solid tumours: an overview. Eur J Intern Med. 2009; 20: 663-671. PMID: 19818284
Non-Patent Document 29: Jarvandi S, Davidson NO, Schootman M. Increased risk of colorectal cancer in type 2 diabetes is independent of diet quality. PLoS One. 2013; 8:e74616. PMID: 24069323
Non-Patent Document 30: Fujihara S, Kato K, Morishita A, et al. Antidiabetic drug metformin inhibits esophageal adenocarcinoma cell proliferation in vitro and in vivo. Int J Oncol. 2015; 46: 2172-2180. PMID: 25709052
Non-Patent Document 31: Berger NA. Young Adult Cancer: Influence of the Obesity Pandemic. Obesity (Silver Spring). 2018; 26: 641-650. PMID: 29570247
Non-Patent Document 32: Hiwasa T, Shimada H. Autoantibody in Cancer. In: Shimada H, ed. Biomarkers in Cancer Therapy (ISBN: 978-981-13-7295-7), Singapore, Springer Nature. 2018: 25-40. https://doi.org/10.1007/978-981-13-7295-7_3

### Disclosure of the Invention

### Problems to be Solved by the Invention

According to the Vital Statistics (2018) of the Ministry of Health, Labour and Welfare of Japan, cancers, heart diseases, cerebrovascular diseases, and renal failure are ranked as the first, second, fourth, and eighth leading causes of death of Japanese, respectively. Also, senescence, pneumonia, unexpected accidents, aspiration pneumonitis, dementia, and suicide are ranked as the third, fifth, sixth, seventh, ninth, and tenth causes of death of Japanese, respectively. According to the WHO, ischemic heart disease, cerebral stroke, trachea/bronchus/lung cancers, diabetes mellitus, and kidney diseases are ranked as the first, second, fifth, eighth, and ninth leading causes of death in the world (2019). Among these causes of death, cancers, heart diseases, cerebrovascular diseases, and renal failure, and diabetes mellitus which is a risk factor for such organic diseases, are conditions whose onset and progress can be prevented by health administration such as early diagnosis. Thus, if appropriate treatment, therapy, daily life guidance, etc. with respect to these conditions can be conducted by use of disease-specific markers, it can make a significant contribution to promotion of human health.

In the coronavirus (COVID-19) pandemic of 2020, the presence of pre-existing condition (e.g., diabetes mellitus, heart diseases, cerebrovascular diseases, cancers, and kidney diseases) is thought to have been one key factor for aggravation of COVID-19. Needless to say, such pre-existing conditions are also general factors for aggravation of infections other than COVID-19. Thus, there is keen demand for provision of a biomarker which can easily detect the presence of such a pre-existing condition, which may cause aggravation of such infections.

Under such circumstances, the related art will further be reviewed. The serum levels of the aforementioned humoral antibodies (i.e., ATP2B4, BMP-1, and DHPS), serving as markers with respect to atherosclerosis, were found to rise also in samples of esophageal cancer patients (see Non-Patent Documents 6 and 7). That is, "artery lesions" typically including arteriosclerosis (e.g., atherosclerosis) can conceivably promote carcinogenesis. Actually, "angiogenesis," which is one example of "artery lesions," is an essential factor for the progress of solid cancer (see Non-Patent Document 28), and diabetes mellitus and obesity, which may induce arteriosclerosis, are risk factors for colorectal cancer and esophageal cancer (see Non-Patent Documents 29, 30, and 31). Meanwhile, since internal organs interact with one another to a greater or lesser extent (see Non-Patent Document 32), detection of "artery lesions" such as arteriosclerosis and angiogenesis may capture symptoms of cerebrovascular and cardiovascular disorders, diabetes mellitus, kidney diseases, solid cancers, etc., thereby possibly leading to early detection of such diseases. If these life-threatening diseases can be detected in an early stage, or symptoms thereof can be detected, by use of a single biomarker, the value of the biomarker would be highly significant.

In addition, it would be remarkably significant to provide means for easily determining, by use of a single biomarker, the presence of a pre-existing condition, which may cause aggravation of infections.

### Means for Solving the Problems

The present inventors have found that the presence of an artery lesion is detected by determining a rise in the level of an antibody to protein KIAA0513 in body fluid, whereby data showing the symptoms or presence of a disease involving the artery lesion can be acquired. The inventors have also found that data showing the potential or actual presence of cerebrovascular or cardiovascular disorder, diabetes mellitus, chronic kidney disease, or a solid cancer can also be acquired by determining a rise in the aforementioned humoral antibody level. The inventors have further found that the data showing the potential or actual presence of cerebrovascular or cardiovascular disorder, diabetes mellitus, chronic kidney disease, or a solid cancer may also be employed as data showing the presence of an artery lesion involved in the diseases. The inventors have yet further found that data showing the symptoms or presence of a disease involving an artery lesion, or data showing the potential or actual presence of cerebrovascular or cardiovascular disorder, diabetes mellitus, chronic kidney disease, or a solid cancer are acquired by detecting the presence of an artery lesion based on a rise in the level of an antibody to protein KIAA0513 in body fluid as an index, whereby the presence of a pre-existing condition which is a factor for aggravation of infections can be determined.

Accordingly, in a first aspect of the present invention, there is provided a method for acquiring data, the method comprising: determining a level of an antibody to a protein having an amino acid sequence represented by SEQ ID NO: 1 or 2 which forms protein KIAA0513, or a portion of the protein, or to a protein having an amino acid sequence represented by SEQ ID NO: 1 or 2 in which 10% or less of the amino acid residues (the decimal point being suppressed) are deleted, substituted, or added, or a portion of the protein, the antibody being potentially present in a body fluid sample collected from a living body; and acquiring data showing a rise in the antibody level as data showing the presence of an artery lesion (hereinafter, the method may also be referred to as the "data acquisition method of the present invention 1") .

In a second aspect of the present invention, there is provided a method for acquiring data, the method comprising: determining a level of an antibody to a protein having an amino acid sequence represented by SEQ ID NO: 1 or 2 which forms protein KIAA0513, or a portion of the protein, or to a protein having an amino acid sequence represented by SEQ ID NO: 1 or 2 in which 10% or less of the amino acid residues (the decimal point being suppressed) are deleted, substituted, or added, or a portion of the protein, the antibody being potentially present in a body fluid sample collected from a living body; and acquiring data showing a rise in the antibody level as data showing the potential or actual presence of cerebrovascular or cardiovascular disorder, diabetes mellitus, chronic kidney disease, or a solid cancer (hereinafter, the method may also be referred to as the "data acquisition method of the present invention 2").

The data showing the onset of the diseases which data are obtained through the data acquisition method of the present invention 2 may also be employed as data showing the presence of an artery lesion involved in the diseases.

Unless otherwise specified, the expression "the data acquisition method of the present invention" in the present specification collectively refers to the data acquisition methods of the present invention 1 and 2.

Meanwhile, protein KIAA0513 is known to include three splicing variants: isoform c (301 amino acids, NP_001284695.1: SEQ ID NO: 1), isoform b (301 amino acids, NP_001273495.1), and isoform a (411 amino acids, NP_055547.1: SEQ ID NO: 2).

The 301 full-length amino acids of KIAA0513 isoforms c or b completely coincide with the initial 301 amino acids of KIAA0513 isoform a. The nucleotide sequence of an mRNA precursor encoding KIAA0513 isoform c differs from that encoding isoform b. However, the amino acid sequence of protein KIAA0513 formed via expression of mature mRNA formed through splicing as a template is common. Thus, in the present specification, without being particularly specified, the expression "protein KIAA0513" refers to protein KIAA0513 isoforms b and/or c (SEQ ID NO: 1). A particular specification is given, in the case where "protein KIAA0513" is intended to refer to isoform a.

Additionally, the present inventors have obtained purified GST-KIAA0513 isoforms c and a, and have investigated the serum level of an antibody to protein KIAA0513 in serum samples of healthy donors (HD), acute ischemic stroke (AIS) patients, and cardiovascular disorder (CVD) patients (Example 6) .

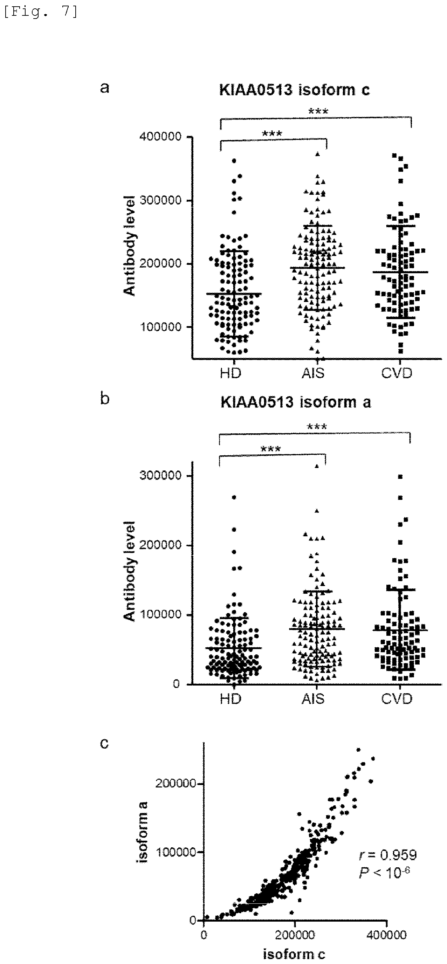

As a result, they have found that both KIAA0513 isoforms c and a exhibit a higher serum antibody level in AIS patients and CVD patients, as compared with in HDs (Figs. 7a and b). The reactivity of KIAA0513 isoform c is slightly higher than that of KIAA0513 isoform a. However, the relative reactivity values closely correlate with each other (Fig. 7c).

The test results have revealed that the main epitope site of a humoral antibody to protein KIAA0513 is present in KIAA0513 isoform c and the initial 301 amino acids of KIAA0513 isoform b. Therefore, "a portion of protein KIAA0513" for use in detection of the humoral antibody to protein KIAA0513 based on antigen-antibody reaction preferably includes the aforementioned overlapping (common) amino acid sequence of the KIAA0513 isoforms c, b, and a.

The data acquisition method of the present invention may also be expressed as, for example, "a method for detecting an artery lesion, the method comprising: determining a level of an antibody to a protein having an amino acid sequence represented by SEQ ID NO: 1 or 2 which forms protein KIAA0513, or a portion of the protein, or to a protein having an amino acid sequence represented by SEQ ID NO: 1 or 2 in which 10% or less of the amino acid residues (the decimal point being suppressed) are deleted, substituted, or added, or a portion of the protein, the antibody being potentially present in a body fluid sample collected from a living body; and detecting an artery lesion based on a rise in the antibody level as an index," and a method for diagnosing or detecting cerebrovascular or cardiovascular disorder, diabetes mellitus, chronic kidney disease, or a solid cancer, the method comprising: determining a level of an antibody to a protein having an amino acid sequence represented by SEQ ID NO: 1 or 2 which forms protein KIAA0513, or a portion of the protein, or to a protein having an amino acid sequence represented by SEQ ID NO: 1 or 2 in which 10% or less of the amino acid residues (the decimal point being suppressed) are deleted, substituted, or added, or a portion of the protein, the antibody being potentially present in a body fluid sample collected from a living body; and diagnosing or detecting cerebrovascular or cardiovascular disorder, diabetes mellitus, chronic kidney disease, or a solid cancer based on a rise in the antibody level as an index."

The present invention also provides a kit for acquiring data, the kit being employed in carrying out the data acquisition method 1 or 2 of the present invention (hereinafter, the kit may also be referred to simply as the "kit of the present invention").

The data acquiring kit of the present invention may also be expressed as, for example, "a kit for carrying out a method for detecting an artery lesion, the method comprising: determining a level of an antibody to a protein having an amino acid sequence represented by SEQ ID NO: 1 or 2 which forms protein KIAA0513, or a portion of the protein, or to a protein having an amino acid sequence represented by SEQ ID NO: 1 or 2 in which 10% or less of the amino acid residues (the decimal point being suppressed) are deleted, substituted, or added, or a portion of the protein, the antibody being potentially present in a body fluid sample collected from a living body; and detecting an artery lesion based on a rise in the antibody level as an index," and "a kit for carrying out a method for diagnosing or detecting cerebrovascular or cardiovascular disorder, diabetes mellitus, chronic kidney disease, or a solid cancer, the method comprising: determining a level of an antibody to a protein having an amino acid sequence represented by SEQ ID NO: 1 or 2 which forms protein KIAA0513, or a portion of the protein, or to a protein having an amino acid sequence represented by SEQ ID NO: 1 or 2 in which 10% or less of the amino acid residues (the decimal point being suppressed) are deleted, substituted, or added, or a portion of the protein, the antibody being potentially present in a body fluid sample collected from a living body; and diagnosing or detecting cerebrovascular or cardiovascular disorder, diabetes mellitus, chronic kidney disease, or a solid cancer based on a rise in the antibody level as an index."

### Effects of the Invention

According to the present invention, firstly, data showing the presence of an artery lesion can be acquired. Based on the data, the symptoms and presence of a disease involving the artery lesion can be grasped. Through taking measures including daily-life guidance and medication on the basis of the data, promotion of health as well as prevention/treatment of diseases can be successfully achieved. Secondarily, there can be acquired data showing the potential or actual presence of cerebrovascular or cardiovascular disorder, diabetes mellitus, chronic kidney disease, or a solid cancer. Based on the data, the onset of the aforementioned grave diseases can be predicted, and these diseases can be detected in an early stage. Through taking measures including daily-life guidance and medication on the basis of the data, prevention/treatment of diseases can be successfully achieved.

The present invention further provides basic data for determining the presence of a pre-existing condition, which is a factor for aggravation of infections.

### Brief Description of the Drawings

[Fig. 1]

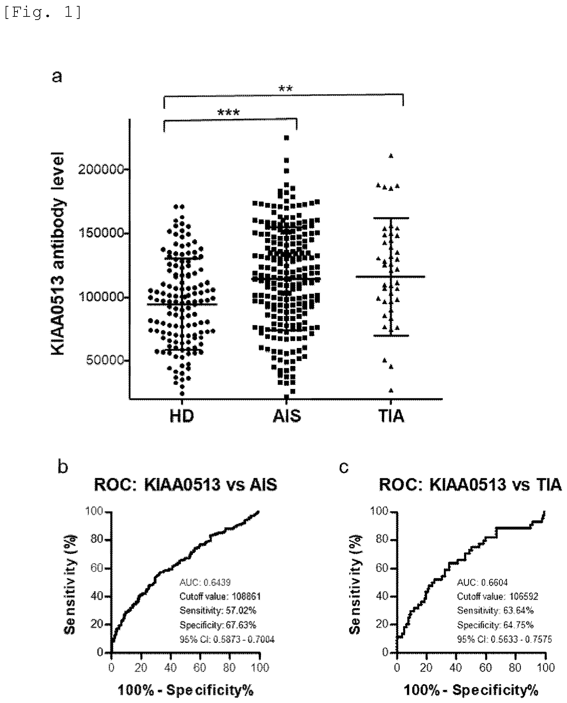

   Fig. 1 shows the results of determination of serum KIAA0513 antibody levels in healthy donors (HD), acute ischemic stroke (AIS) patients, and transient ischemic attack (TIA) patients through the AlphaLISA method. Graph a is a distribution diagram showing the results when protein KIAA0513 is used as an antigen. The vertical axis of the diagram indicates alpha counts corresponding to the antibody level. The bars of each segment of the diagram represent an average value and a value of average and ±SD. Graphs b and c show the results of ROC analysis. In each ROC diagram, there are provided AUC value, cut-off value, sensitivity, specificity, and 95% confidence interval.
[Fig. 2]

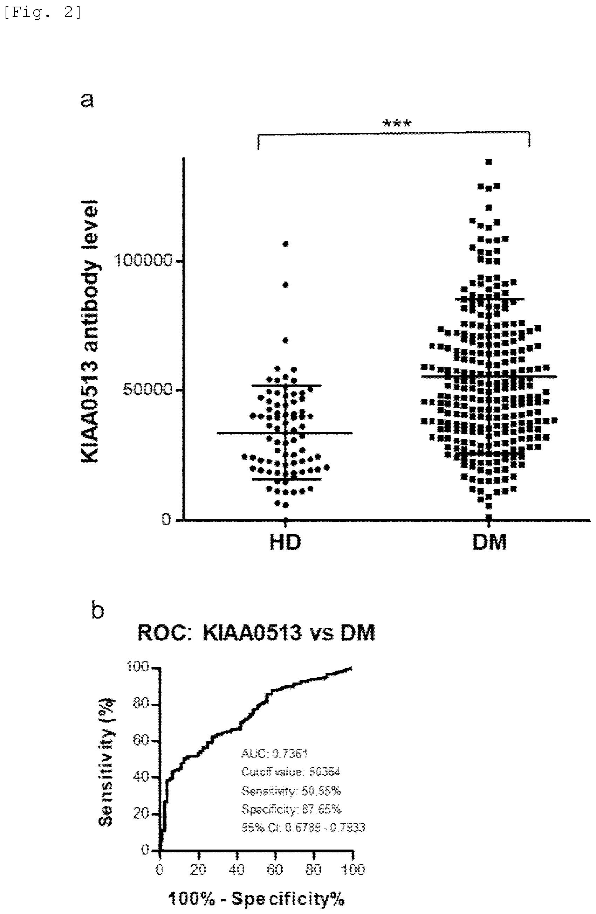

   Fig. 2 shows the results of determination of serum KIAA0513 antibody levels in HDs and diabetes mellitus (DM) patients through the AlphaLISA method. Graph a is a distribution diagram showing the results when protein DIDO1 is used as an antigen. Graph b shows the results of ROC analysis. The legends for the distribution diagram and ROC diagram are the same as those for Fig. 1.
[Fig. 3]

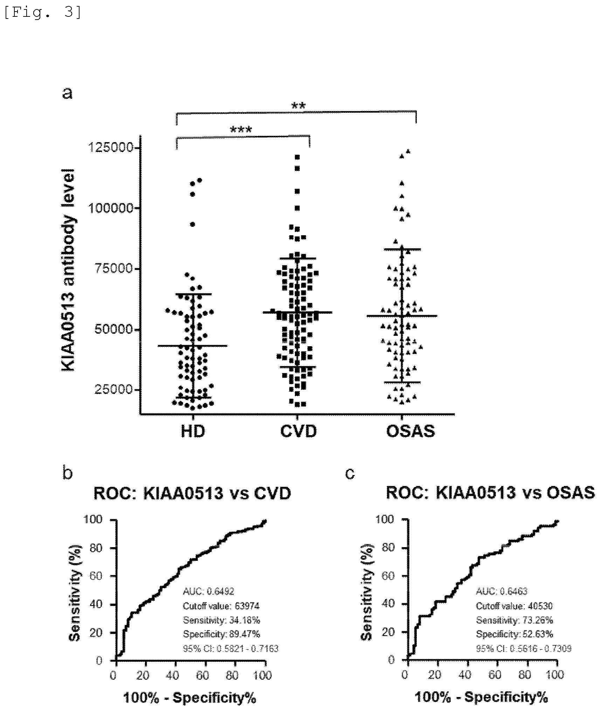

   Fig. 3 shows the results of determination of serum KIAA0513 antibody levels in HDs, cardiovascular disorder (CVD) patients, and obstructive sleep apnea syndrome (OSAS) patients through the AlphaLISA method. Graph a is a distribution diagram, and Graphs b and c show the results of ROC analysis. The legends for the distribution diagram and ROC diagrams are the same as those for Fig. 1.
[Fig. 4]

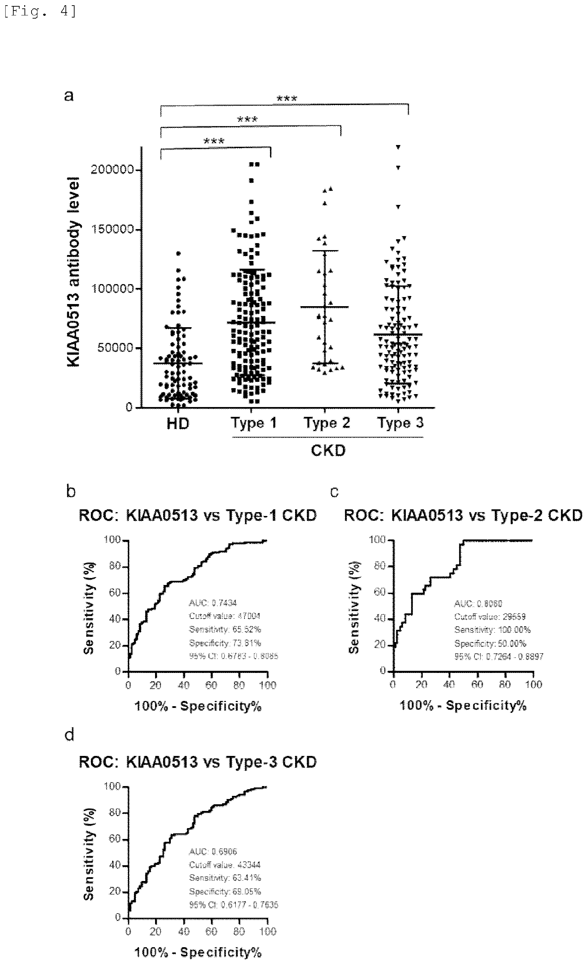

   Fig. 4 shows the results of determination of serum KIAA0513 antibody levels in HDs and chronic kidney disease (CKD) patients through the AlphaLISA method, when protein KIAA0513 was used as an antigen. Graph a is a distribution diagram, and Graphs b, c, and d show the results of ROC analysis. The legends for the distribution diagram and ROC diagrams are the same as those for Fig. 1.
[Fig. 5]

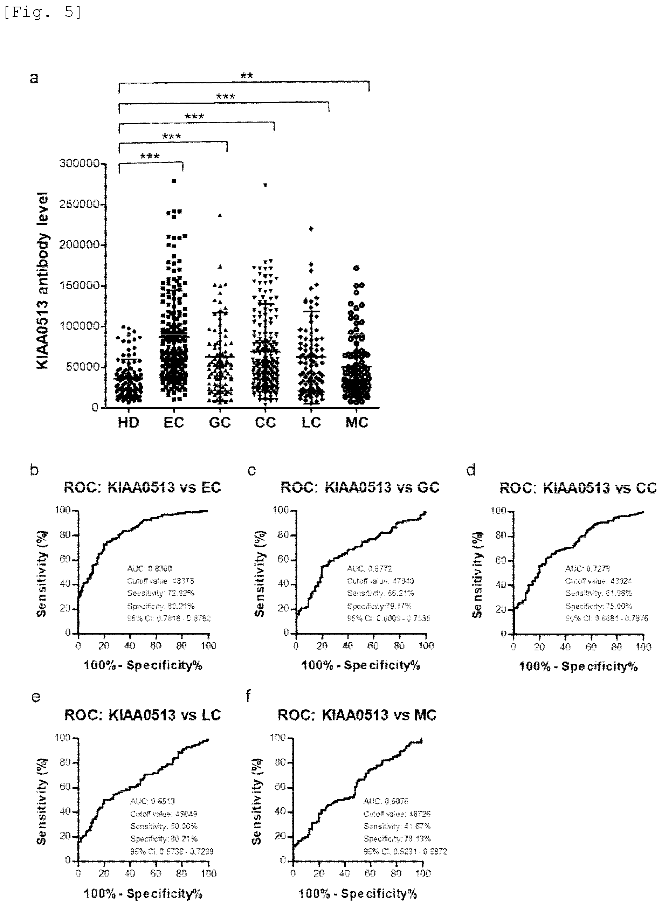

   Fig. 5 shows the results of determination of serum KIAA0513 antibody levels in HDs and cancer patients through the AlphaLISA method. The serum antibody levels are determined in HDs and cancer patients (esophageal cancer (EC), gastric cancer (GC), colorectal cancer (CC), lung cancer (LC), and mammary cancer (MC)), when protein KIAA0513 is used as an antigen. Graph a is a distribution diagram, and Graphs b to f show the results of ROC analysis. The legends for the distribution diagram and ROC diagrams are the same as those for Fig. 1.
[Fig. 6]

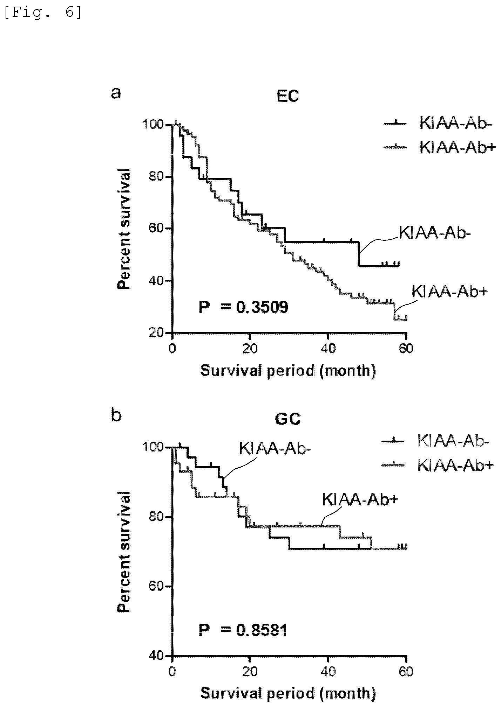

   Fig. 6 shows comparison in percent survival after surgery of esophageal cancer (EC) and gastric cancer (GC), with cases of KIAA0513 antibody positive and negative. Percent survival values after surgery are compared through Kaplan-Meyer plotting.
[Fig. 7]
   Fig. 7 shows comparison in serum antibody level when KIAA0513 isoforms c and a are used as antigens. Graphs are distribution diagrams showing serum antibody levels in HDs, AIS patients, and CVD patients, with respect to KIAA0513 isoform c (Graph a) and KIAA0513 isoform a (Graph b). Graph c is a correlation diagram showing antibody levels (alpha counts) in samples, when KIAA0513 isoforms c and a are used an antigen, respectively.

### Modes for Carrying Out the Invention

### (1) Data acquisition method of the present invention

### (a) Data to be acquired

The data acquired through the data acquisition method of the present invention 1 are "data showing the presence of an artery lesion." The artery lesion corresponds to abnormality in the structure and components of an artery, and modes thereof include occlusion, expansion, and fistulation. The progress of artery lesion involves arteriosclerosis (atherosclerosis, arteriolosclerosis, or medial sclerosis), anomalous angiogenesis in chronic inflammation or solid cancer, and other conditions. In many cases, arteriosclerosis is known to be triggered by progress of diabetes mellitus and obesity. Particularly, cerebrovascular and cardiovascular disorders, chronic kidney disease, aortic aneurysm, retinopathy, feet gangrene, etc. are known to be triggered by progress of arteriosclerosis, in particular, atherosclerosis. Diabetes mellitus and obesity are also risk factors for solid cancers (colorectal cancer, esophageal cancer, etc.). Thus, the "data showing the presence of an artery lesion" obtained by the data acquisition method of the present invention 1 may be employed as data showing the possibility of onset or progress of diabetes mellitus, arteriosclerosis, solid cancer, etc. In addition, the data may also be employed as data showing the potential or actual presence of severe circulatory diseases such as cerebrovascular and cardiovascular disorders, chronic kidney disease, aortic aneurysm, retinopathy, and feet gangrene.

The data acquired through the data acquisition method of the present invention 2 are "data showing the potential or actual presence of diabetes mellitus, cerebrovascular or cardiovascular disorder, chronic kidney disease, or solid cancers." Examples of the cerebrovascular or cardiovascular disorder include cerebral infarction, cerebral stroke, myocardial infarction, and angina pectoris. Examples of the solid cancer include esophageal cancer, gastric cancer, colorectal cancer, lung cancer, and mammary cancer. The rise in the level of the humoral antibody to protein KIAA0513 can be employed as reliable data showing the potential or actual presence of esophageal cancer, colorectal cancer, gastric cancer, or lung cancer, particularly esophageal cancer. The data acquisition method of the present invention 2 often overlaps with the data acquisition method of the present invention 1.

For example, the onset of any of the aforementioned diseases in a subject can be accurately predicted by periodically conducting the data acquisition method of the present invention 1 or 2 on the patient, including monitoring of the level of the humoral antibody to "protein KIAA0513." Particularly, once a subject has developed a severe circulatory disease such as cerebral infarction or myocardial infarction or a solid cancer, the life of the subject is endangered. Even if death is avoided, the subject may suffer a severe sequela. Therefore, prediction of the onset of such diseases is the best solution, and a humoral anti-KIAA0513 antibody marker may be employed as a biomarker for acquiring data concerning prediction of the onset.

When epidemic of a specific infection is recognized, the presence of a pre-existing condition, which is a factor for aggravation of the infection, is determined on the basis of the data acquired through the data acquisition method of the present invention 1 or 2. As a result, the patient suffering the infection can be more suitably treated. In the case where the obtained data indicate the presence of, for example, an artery lesion, the patient contracting the infection is preferentially hospitalized so as to prevent aggravation. In the case where the data indicate no artery lesions, the subject is made to stay at home, whereby hospital capacity including medical staff, beds, etc. can be effectively furnished. In this case, the infected subjects can be appropriately treated in accordance with their degree of aggravation risk. Thus, the risk of medical care breakdown, which would otherwise be caused by shortage of medical staff and beds due to epidemic of infection, can be reduced.

The aforementioned infection includes various viral diseases, bacterial diseases, etc. No particular limitation is imposed on the infection, so long as the presence of a pre-existing condition is an aggravation factor of the infection. Particularly in the case of infections in the respiratory system, which occasionally cause outbreak, the aggravation risk of the infections is desirably estimated by mass examination (screening). The present invention, which can be implemented in a simple mode (e.g., immunochromatography), is particularly suited for such mass examination. Examples of the aforementioned infections include, but are not limited to, respiratory system infections such as infections with beta-coronaviruses (for example, COVID-19, SARS, and MARS); infections with influenza viruses; and infections with RS virus, adenovirus, rhinovirus, and the like.

### (b) Body fluid sample collected from living body

The term "body fluid" of "a body fluid sample collected from a living body" which is a target for data acquisition in the data acquisition method of the present invention, includes blood, lymph, or the like. The term "sample" refers to body fluid itself as collected from a body, or a processed product of the fluid. Among such body fluid samples, a blood sample is preferred. Examples of the blood sample include whole blood samples, serum samples, and plasma samples. Of these, serum samples and plasma samples are preferred, with serum samples being particularly preferred. The blood samples may undergo anti-clotting treatment (e.g., treatment with heparin) in advance.

### (c) Humoral antibody-capturing antigen (KIAA0513 antigen)

In the data acquisition method of the present invention, protein KIAA0513 having an amino acid sequence represented by SEQ ID NO: 1 or 2 is the basis for capturing a humoral antibody. The entirety or a portion of protein KIAA0513 may be used as a humoral antibody-capturing antigen. Furthermore, in order to establish a more efficient detection system, a shorter chain amino acid sequence (peptide) may be selected. In this case, the number of amino acids of the amino acid sequence is about 7 to about 30, more preferably about 10 to 20.

In the entirety or a portion of any of the amino acid sequences, 10% or less of the amino acid residues (the decimal point being suppressed) may undergo modification (deletion, substitution, or addition), so long as the target humoral antibody (i.e., a humoral antibody to protein KIAA0513 or to a peptide having a partial sequence thereof) can be captured. In other words, when the amino acid-modified antigen candidate for capturing a target humoral antibody is used for capturing the humoral antibody through, for example, a technique similar to the technique described in the Examples of the present specification, data concerning the target artery lesion or disease must be acquired on the basis of the level of the captured antibody.

The term "deleting" refers to deletion of any amino acid residue in the relevant amino acid sequence. The amino acid residue at the N-terminal side of the deleted amino acid residue and that at the C-terminal side of the deleted amino acid residue are linked via a peptide bond. In the case of deletion of the N-terminal amino acid residue or the C-terminal amino acid residue, no linkage is present. The number of deleted residues is counted as "the number of amino acid deletions." The term "substituting" refers to substitution of any amino acid residue in the relevant amino acid sequence, "with another amino acid residue." The new amino acid residue is linked to the amino acid residue at the N-terminal side thereof and that at the C-terminal side thereof via a peptide bond. In the case of substitution of the N-terminal side or C-terminal side amino acid residue, the amino acid residue is linked via a peptide bond to another C-terminal side or N-terminal side amino acid residue. The number of substituted residues is counted as "the number of amino acid substitutions." The term "adding" refers to addition of one or more new amino acid residues to one or more peptide bond sites in the relevant amino acid sequence, to thereby form a new peptide bond(s). Addition of an amino acid residue to the C terminal or the N terminal is also encompassed in the concept "addition." The number of added substituted amino acid residues is counted as "the number of amino acid additions." Hereinafter, "a protein having an amino acid sequence represented by SEQ ID NO: 1 or 2 which forms protein KIAA0513, or a portion of the protein, or a protein having an amino acid sequence represented by SEQ ID NO: 1 or 2 in which 10% or less of the amino acid residues (the decimal point being suppressed) are deleted, substituted, or added, or a portion of the protein" is also referred to as a "KIAA0513 antigen."

The KIAA0513 antigen having a specific amino acid sequence may be obtained through a customary method. In one specific procedure, based on the nucleotide sequence of the KIAA0513 gene represented by SEQ ID NO: 1 or 2, nucleic acid amplification primers for amplifying a double-strand DNA including the entirety or a portion of nucleotide sequence are designed. Through PCR or a similar technique employing the amplification primers, a gene amplification product is yielded as the entirety or a portion of the KIAA0513 gene. The amplification product is incorporated into a prokaryotic cell expression vector (e.g., pGEX-4T), and a transformant including the vector is selected. Expression of the entirety or a portion of the KIAA0513 gene is induced by adding a chemical agent such as IPTG (isopropyl-β-D-thiogalactoside). The entirety or a portion of the thus-formed protein KIAA0513 may be purified through, for example, affinity chromatography by use of glutathione-Sepharose (product of GE Healthcare Life Sciences).

If needed, a modification structure may be appropriately added to the protein or the peptide used as such a KIAA0513 antigen. For example, as mentioned hereinbelow, there may be employed a GST (glutathione S-transferase)-fused protein or peptide, a biotin-fused protein or peptide, etc., which are used in AlphaLISA (Amplified Luminescence Proximity Homogeneous Assay-Linked ImmunoSorbent Assay).

### (d) Specific procedure of data acquisition

According to the data acquisition method of the present invention, the level of an antibody to the KIAA0513 antigen in a body fluid sample (e.g., a blood sample) collected from a living body is determined. When the determined value is greater than a standard value (cut-off value), the increase in KIAA0513 antigen level in the body fluid sample donor is confirmed. The data can be employed as an index for showing the presence of an artery lesion in the body fluid sample donor, or for showing the potential or actual presence of cerebrovascular or cardiovascular disorder, diabetes mellitus, chronic kidney disease, or a solid cancer in the body fluid sample donor.

The standard value of the humoral antibody level with respect to the KIAA0513 antigen in a test sample may be derived through the following procedure. First, a control sample population is established from subjects exhibiting no abnormality (e.g., diabetes, a respiratory disease, or a cancer) in a medical examination (i.e., healthy donors). The level of a humoral antibody to the KIAA0513 antigen is determined, with respect to the test samples obtained from the control sample population. The determination data are statistically processed, to thereby obtain an average, a standard deviation, etc., and derive the standard value including the cut-off value, on the basis of the data.

In one possible embodiment of the humoral antibody level determination, the KIAA0513 antigen which is immobilized on a substrate is brought into contact with a body fluid sample collected from a living body. Bonding (based on antigen-antibody reaction) of a humoral antibody to the KIAA0513 antigen in the body fluid sample collected from the living body is detected as a signal. More specifically, the determination can be performed through immunochromatography, AlphaLISA, ELISA, indirect immunofluorescence, Western blotting (immunoblotting), turbidimetry, nephelometry, latex-coagulation turbidimetry, CLEIA, or the like. All the exemplified determination techniques have been established for determining the level of a target substance (antibody) in a body fluid sample collected from a living body.

In one mode of the immuno-chromatographic method, a body fluid sample and a buffer liquid containing a gold colloid-bound anti-human IgG antibody are added to a cellulose membrane onto which the KIAA0513 antigen has been applied, and the liquid mixture is slowly moved on the membrane. If an antibody to the KIAA0513 antigen is present in the body fluid sample, a line is formed at a position where the antigen is applied on the surface of the membrane. The line can be visually observed. Since the immuno-chromatographic method can be conducted in a simpler manner, as compared with other assay means, the method is particularly suited for mass examination. In other words, when the data acquisition method of the present invention is carried out through the immuno-chromatographic method, desired disease-related data can be acquired in a short period of time (about 10 minutes) even in small facilities (for example, a clinic), a public health center, and other facilities.

In the case of AlphaLISA, when a GST-fused KIAA0513 antigen is used as an antigen for capturing a humoral antibody, glutathione-conjugated donor beads are used. The antigen for capturing a humoral antibody, a blood sample, and acceptor beads to which an anti-human IgG antibody has been bound are mixed together, and the mixture is incubated at room temperature for several hours to several days, to thereby form an antigen-antibody complex. The complex is irradiated with light (680 nm), and the resultant light having a wavelength of 520 to 620 nm is detected, whereby the target humoral antibody can be quantitated. In indirect immunofluorescence assay, a body fluid sample collected from a living body is brought into contact with a protein array in which the KIAA0513 antigen has been immobilized on a substrate, to thereby form a complex of the KIAA0513 antigen with an anti-KIAA0513 humoral antibody. Then, a fluorescent-labeled secondary antibody is brought into contact with the humoral antibody complex, whereby a humoral antibody to protein KIAA0513 can be quantitated. In ELISA, the same secondary antibody as employed in indirect immunofluorescence assay is used, except that the label is changed to an enzyme, to thereby conduct quantitation. The label for the secondary antibody can be selected from various enzymes. In Western blotting, a KIAA0513 antigen is subjected to electrophoresis by use of SDS-polyacrylamide gel, and the resultant band is transferred to a carrier such as a nitrocellulose membrane. Then, a body fluid sample collected from a living body is brought into contact with the carrier, to thereby form a (KIAA0513 antigen)-(anti-KIAA0513 humoral antibody) complex. Detection of the complex is performed by use of a secondary antibody, to thereby achieve quantitation. In turbidimetry or nephelometry, a body fluid sample collected from a living body is brought into contact with the KIAA0513 antigen, to thereby form a (KIAA0513 antigen)-(anti-KIAA0513 humoral antibody) complex. Detection of the complex is performed on the basis of change in turbidity (in the case of turbidimetry) or scattered light (in the case of nephelometry), to thereby achieve quantitation. In latex agglutination turbidimetry, a body fluid sample collected from a living body is brought into contact with latex particles to which the KIAA0513 antigen has been bound, to thereby form agglutinated latex particles via interaction between the humoral anti-KIAA0513 antibodies bounded to latex particles, and the thus-formed agglutinated latex particles are quantitated. In one possible mode of CLEIA assay, a body fluid sample collected from a living body is brought into contact with magnetic particles to which the KIAA0513 antigen has been bound, to thereby form, on the surfaces of the magnetic particles, a (KIAA0513 antigen)-(anti-KIAA0513 antibody) complex. Unreacted matter is removed through collecting magnetism, and the product is subjected to an appropriate fluorescent chemical treatment or the like. Thus thus-treated complex is quantitated.

### (2) Kit of the present invention

An embodiment of the kit of the present invention employs AlphaLISA as an assay means and has a set including a GST-fused KIAA0513 antigen, glutathione-conjugated donor beads, and anti-human IgG-conjugated acceptor beads.

Another embodiment of the kit of the present invention employs ELISA as an assay means and has a set including a plate on which the aforementioned KIAA0513 antigen has been immobilized, a labeled secondary antibody to the anti-KIAA0513 antibody, and a reagent for developing the label of the secondary antibody.

Still another embodiment of the kit of the present invention employs latex agglutination turbidimetry and includes latex particles on which the aforementioned KIAA0513 antigen has been immobilized.

Yet another embodiment of the kit of the present invention employs CLEIA and includes magnetic particles on which the aforementioned KIAA0513 antigen has been immobilized, a labeled secondary antibody with respect to the anti-KIAA0513 antibody, and a reagent for developing the label of the secondary antibody.

Notably, the aforementioned embodiments of the kit are illustrated merely as examples, and kits employing other assay means are also encompassed in the scope of the kit of the present invention. Furthermore, the number of the aforementioned kit elements may be further reduced so as to encourage self-supply or out-sourcing of medical tests. In contrast, elements such as diluent and reagent tubes are added to the kit so as to realize immediate use of the kit of the present invention. Other additional elements suited for the mode of tests may also be added.

### Examples

Examples of the present invention will next be described.

### [Production Examples and Analytical Techniques]

### <Antigen proteins employed in the Examples>

### (1) Identification of antigen protein

In the Examples, by means of a human protein microarray (ProtoArray (registered trademark), product of Thermo Fisher Scientific), proteins KIAA0513 were identified as antigens which are recognized by a serum antibody of atherosclerosis patients.

In a specific procedure, the N-terminal of "KIAA0513 isoform a" (SEQ ID NO: 2) was modified with GST. The full-length region of 411 amino acids, or a portion of 301 amino acids from the N-terminal was used. Notably, the latter "portion of 301 amino acids from the N-terminal" corresponds to KIAA0513 isoform c (SEQ ID NO: 1).

### (2) Preparation of antigen proteins (Formation and purification of proteins KIAA0513)

Human proteins KIAA0513 were prepared through the following procedure.

Each of the cDNA fragments encoding human KIAA0513 isoforms a and c was inserted into an *Eco*RI/*Sal*I site of a plasmid vector pGEX-4T-1 (GE Healthcare Life Sciences, Pittsburgh, PA), in which a glutathione S-transferase (GST) gene site is provided on the upstream side of the cloning site, to thereby produce recombinant plasmids pGEX-4T-1-KIAA0513 (isoform a) and pGEX-4T-1-KIAA0513 (isoform c) for gene expression, respectively. Each plasmid was incorporated into *E. coli* BL-21 for transformation, and expression of the corresponding cDNA encoding protein KIAA0513 was induced through a treatment with 0.1mM IPTG (isopropyl-β-D-thiogalactoside) at 25°C for 4 hours. Thereafter, pellets of *E. coli* were recovered and dissolved by use of BugBuster Master Mix (Merck Millipore, Darmstadt, Germany), to thereby yield a protein extract. Each of the GST-protein KIAA0513 isoforms a and c present in the protein extract was purified through column chromatography with glutathione-Sepharose (GE Healthcare Life Sciences). Through changing the buffer to PBS, the aforementioned "proteins KIAA0513" were obtained. GST as a control was purified in the same manner.

### <Analysis through AlphaLISA>

Prior to disclosure of specific working examples, the analysis through AlphaLISA employed in the Examples will next be outlined.

Specifically, the AlphaLISA (Amplified Luminescence Proximity Homogeneous Assay) was performed by use of a 384-well microtiter plate (white opaque OptiPlate^{™}, Perkin Elmer, Waltham, MA). To each well, a serum (2.5 µL) 100-fold diluted with AlphaLISA buffer (25mM HEPES, pH: 7.4, 0.1% casein, 0.5% Triton X-100, 1-mg/mL dextran-500, 0.05% Proclin-300) was added. GST or protein KIAA0513 (i.e., KIAA0513 isoform a or c) (10 ug/mL) (antigen), diluted with AlphaLISA buffer, was mixed with the serum in each well. The mixture was incubated at room temperature for 6 to 8 hours. Separately, anti-human IgG-conjugated acceptor beads (2.5 µL at 40 µg/mL), and glutathione- or streptavidin-conjugated donor beads (2.5 µL at 40 µg/mL) were diluted with AlphaLISA buffer, and the thus-diluted product was mixed with the incubated product. The resultant mixture was allowed to stand at room temperature for 1 to 14 days. Thereafter, generated photons were counted by means of EnSpire Alpha microplate reader (Perkin Elmer), to thereby determine "Alpha Count." In the case of protein KIAA0513, a control GST value was subtracted, whereas in the case of the biotinylated peptide, a control buffer value was subtracted, whereby the antibody level specific to each KIAA0513 antigen was determined.

### [Example 1]

### Studies of biomarker in transient ischemic attack (TIA) patients and acute ischemic stroke (AIS) patients through AlphaLISA

Acute ischemic stroke (AIS) refers to a pathological condition in which a patient receives sudden damage in motor function or sensory function due to stagnation of blood flow in the brain. Transient ischemic attack (TIA) refers to a disease in which cerebral nerve cells are damaged through the same mechanism as that of cerebral infarction, but the damage is restored within 24 hours, generally several minutes. In both cases, blood serum samples are collected from relevant patients ≤2 weeks after the onset thereof.

The serum antibody (s-KIAA0513-Abs: serum KIAA0513 antibody) level with respect to KIAA0513 isoform c (SEQ ID NO: 1) was determined in AIS patients and TIA patients. Serum samples of healthy donors (HDs), AIS patients, and TIA patients were collected from Chiba Prefectural Sawara Hospital. In AlphaLISA, the s-KIAA0513-Ab level was found to be significantly higher in TIA patients and AIS patients than in HDs (see Fig. 1).

When the cut-off value was set to "the average + 2SD for HD samples," the s-KIAA0513-Ab positive ratios of HDs, AIS patients, and TIA patients were 0.0%, 7.6%, and 15.6%, respectively (see Table 1).

Through ROC analysis, area under the curve (AUC) values of s-KIAA0513-Abs were found to be 0.6439 [95% confidence interval (CI) = 0.5873-0.7004] with respect to AIS (see Fig. 1b), and 0.6604 (95% CI = 0.5633-0.7575) with respect to TIA (see Fig. 1c).

Thus, TIA, which is a prodromal stage of AIS, was also found to positively correlate with the serum KIAA0513 antibody level. Therefore, the s-KIAA0513-Ab level served as a marker effectively predicting the onset of AIS.

**[Table 1]**

| Sample information | | HD | AIS | TIA |
|---|---|---|---|---|
| | Total sample number | 139 | 228 | 44 |
| | Male/Female | 87152 | 129199 | 24120 |
| | Age (Average ± SD) | 51.6 ± 12.8 | 77.0 ± 11.1 | 68.5 ± 12.1 |

| Subject group | Type of value | KIAA0513-Ab | | |
|---|---|---|---|---|
| HD | Average | 92,849 | | |
| | SD | 36,115 | | |
| | Cutoff value | 165,080 | | |
| | Positive number | 0 | | |
| | Positive (%) | 0.0% | | |
| AIS | Average | 110,913 | | |
| | SD | 40,351 | | |
| | Positive number | 11 | | |
| | Positive (%) | 7.6% | | |
| | P (AIS vs HD) | **<0.001** | | |
| TIA | Average | 122,303 | | |
| | SD | 42,845 | | |
| | Positive number | 5 | | |
| | Positive (%) | **15.6%** | | |
| | *P* (TIA vs HD) | **<0.01** | | |

### [Example 2]

### Studies of biomarker in diabetes mellitus (DM) patients through AlphaLISA

Next, the s-KIAA0513-Abs level of DM patients was investigated.

Serum samples of HDs and DM patients were obtained from Chiba University Hospital.

The s-KIAA0513-Abs level was found to be significantly higher in DM patients than in HDs (see Fig. 2a).

When the cut-off value was set to "the average + 2SD for HD samples," the s-KIAA0513-Ab positive ratios of HDs and DM patients were 2.5% and 26.5%, respectively (see Table 2).

The ability of the antibody marker to indicate the presence of DM was evaluated through ROC analysis. The area under the curve (AUC) value of s-KIAA0513-Abs was 0.7361, and the values of sensitivity and specificity were 50.55% and 87.65%, respectively (see Fig. 2b).

**[Table 2]**

| Sample information | | HD | DM |
|---|---|---|---|
| | Total sample number | 81 | 275 |
| | Male/Female | 46135 | 158/117 |
| | Type 1 DM/Type 2 DM | - | 26/216* |
| | Age (Average ± SD) | 45.2 ± 11.0 | 63.1 ± 12.0 |

| Subject group | Type of value | KIAA0513-Ab | |
|---|---|---|---|
| HD | Average | 33,698 | |
| | SD | 18,121 | |
| | Cutoff value | 69,941 | |
| | Positive number | 2 | |
| | Positive (%) | 2.5% | |
| DM | Average | 55,282 | |
| | SD | 29,897 | |
| | Positive number | 73 | |
| | Positive (%) | 26.5% | |
| | P (vs HD) | **<0.001** | |

### [Example 3]

### Studies of biomarker in cardiovascular disorder (CVD) patients and obstructive sleep apnea syndrome (OSAS) patients through AlphaLISA

Next, the s-KIAA0513-Abs levels of CVD patients and OSAS patients were investigated.

The CVD patients in Chiba University Hospital included acute myocardial infarction patients and unstable angina patients. Generally, OSAS is known to relate to atherosclerosis and is a high risk factor for acute ischemic stroke (AIS) and CVD. Thus, serum samples of OSAS patients obtained from Chiba University Hospital were also analyzed.

As a result, the s-KIAA0513-Abs level was found to be higher in CVD patients and OSAS patients than in HDs (Fig. 3a). The s-KIAA0513-Abs positive ratios of HDs, CVD patients, and OSAS patients were 5.3%, 10.3%, and 11.6%, respectively (see Table 3).

Through ROC analysis, area under the curve (AUC) values of s-KIAA0513-Abs were found to be 0.6492 (95% CI: 0.5821-0.7163) with respect to CVD (see Fig. 3b), and 0.6463 (95% CI: 0.5616-0.7309) with respect to OSAS (see Fig. 3e).

The P value of s-KIAA0513-Ab with respect to CVD was small (<0.0001), but the P value with respect to OSAS was 0.0013. The data suggest that the correlation between the s-KIAA0513-Ab marker and OSAS is less significant than the correlation between the s-KIAA0513-Ab marker and CVD (see Table 3). Thus, conceivably, OSAS does not correlate directly with the s-KIAA0513-Ab marker, but does indirectly correlate with the marker by the mediation of CVD or the like, which is induced by OSAS.

**[Table 3]**

| Sample information | | HD | CVD | OSAS |
|---|---|---|---|---|
| | Total sample number | 76 | 97 | 86 |
| | Male/Female | 43/33 | 81/16 | 59/27 |
| | Age (Average ± SD) | 45.1 ± 11.5 | 66.3 ± 11.4 | 57.8 ± 12.5 |

| Subject group | Type of value | KIAA0513-Ab | | |
|---|---|---|---|---|
| HD | Average | 43,398 | | |
| | SD | 21,374 | | |
| | Cutoff value | 86,146 | | |
| | Positive number | 4 | | |
| | Positive (%) | 5.3% | | |
| CVD | Average | 57,531 | | |
| | SD | 22,460 | | |
| | Positive number | 10 | | |
| | Positive (%) | **10.3%** | | |
| | *P* (CVD vs HD) | **<0.001** | | |
| OSAS | Average | 55,650 | | |
| | SD | 27,493 | | |
| | Positive number | 10 | | |
| | Positive (%) | **11.6%** | | |
| | P (OSA vs HD) | **<0.01** | | |

### [Example 4]

### Studies of biomarker in chronic kidney disease (CKD) patients through AlphaLISA

Next, the s-KIAA0513-Abs level of CKD patients was investigated. CKD is closely related to atherosclerosis.

The studied CKD patients were divided into three groups.

Specifically, the groups were the diabetic nephropathy group (Type 1), the nephrosclerosis group (Type 2), and the glomerulonephropathy group (Type 3).

Serum samples of CKD patients were obtained from a cohort of Kumamoto Prefecture, and those of HDs were obtained from Chiba University.

The serum s-KIAA0513-Abs level was found to be significantly higher in all the three CKD groups than in HD samples (Fig. 4a). More specifically, the s-KIAA0513-Ab positive ratios of HDs, Type 1 CKD patients, Type 2 CKD patients, and Type 3 CKD patients were 6.1%, 29.0%, 37.5%, and 20.3%, respectively (see Table 4), and the highest positive ratio was identified in serum samples of Type 2 CKD patients.

Through ROC analysis, area under the curve (AUC) values of s-KIAA0513-Abs were found to be 0.7434 (95% CI: 0.6783-0.8085) with respect to Type 1 CKD (see Fig. 4b), 0.8080 (95% CI: 0.7264-0.8897) with respect to Type 2 CKD (see Fig. 4c), and 0.6906 (95% CI: 0.6177-0.7635) with respect to Type 3 CKD (see Fig. 4d).

**[Table 4]**

| Sample information | | HD | Type-1 CKD | Type-2 CKD | Type-3 CKD |
|---|---|---|---|---|---|
| | Total sample number | 82 | 145 | 32 | 123 |
| | Male/Female | 44/38 | 106/39 | 21/11 | 70/53 |
| | Age (Average ± SD) | 44.1 ± 11.2 | 66.0 ± 10.4 | 76.0 ± 9.8 | 62.0 ± 11.7 |

| Alpha analysis (antibody level) | | KIAA0513-Ab | | | |
|---|---|---|---|---|---|
| HD | Average | 36,007 | | | |
| | SD | 28,697 | | | |
| | Cutoff value | 93,400 | | | |
| | Positive No. | 5 | | | |
| | Positive rate (%) | 6.1% | | | |
| Typ-1 CKD | Average | 71,773 | | | |
| | SD | 44,434 | | | |
| | Positive No. | 42 | | | |
| | Positive rate (%) | **29.0%** | | | |
| | P (vs HD) | **<0.001** | | | |
| Type-2 CKD | Average | 84,954 | | | |
| | SD | 47,414 | | | |
| | Positive No. | 12 | | | |
| | Positive rate (%) | **37.5%** | | | |
| | P (vs HD) | **<0.001** | | | |
| Type-3 CKD | Average | 61,443 | | | |
| | SD | 41,110 | | | |
| | Positive No. | 25 | | | |
| | Positive rate (%) | **20.3%** | | | |
| | P (vs HD) | **<0.001** | | | |

### [Example 5]

### Studies of biomarker in cancer patients through AlphaLISA

Atherosclerosis is known to be often related to cancers. Thus, the s-KIAA0513-Abs level in serum samples of esophageal cancer (EC), gastric cancer (GC), colon cancer (CC), lung cancer (LC), and mammary cancer (MC) patients was investigated. The serum samples were obtained from Toho University Medical Center.

As a result, the s-KIAA0513-Abs level was significantly higher in serum samples of all cancer patients, as compared with those of HDs. The highest average value of s-KIAA0513-Ab level was identified in the case of esophageal cancer (see Fig. 5a and Table 5). In ROC analysis, the AUC value was as high as 0.8300 in the case of esophageal cancer (see Figs. 5b to 5f).

Next, correlation of the s-KIAA0513-Ab level with survival rates of esophageal cancer (EC) patients and gastric cancer (GC) patients after surgery was investigated. On the basis of the cut-off value obtained through ROC analysis, analytical samples were divided into an s-KIAA0513-Ab positive group and an s-KIAA0513-Ab negative group (see Figs. 5b and 5c). In the case of esophageal cancer, no significant difference was found in overall survival rate between the s-KIAA0513-Ab positive group and the s-KIAA0513-Ab negative group (P=0.3509), but a tendency of poor prognosis was observed in the antibody positive group between 40 to 60 weeks after surgery (Fig. 6a). In contrast, in the case of gastric cancer, no significant difference was found in survival rate between the s-KIAA0513-Ab positive group and the s-KIAA0513-Ab negative group (Fig. 6b).

**[Table 5]**

| Sample information | | HD | EC | GC | CC | LC | MC |
|---|---|---|---|---|---|---|---|
| | Total sample number | 96 | 192 | 96 | 192 | 96 | 96 |
| | Male/Female | 51145 | 155137 | 68128 | 119/74 | 0/96 | 58/38 |
| | Age (Average ± SD) | 57.9 ± 6.0 | 67.4 ± 9.8 | 68.7 ± 10.6 | 66.7 ± 11.7 | 60.9 ± 13.3 | 68.1 ± 9.6 |

| Subject group | Type of value | KIAA0513-Ab | | | | | |
|---|---|---|---|---|---|---|---|
| HD | Average | 36,199 | | | | | |
| | SD | 23,001 | | | | | |
| | Cutoff value | 82,201 | | | | | |
| | Positive number | 8 | | | | | |
| | Positive (%) | 8.3% | | | | | |
| EC | Average | 87,535 | | | | | |
| | SD | 57,134 | | | | | |
| | Positive number | 86 | | | | | |
| | Positive (%) | 44.8% | | | | | |
| | P (EC vs HD) | **<0.001** | | | | | |
| GC | Average | 62,714 | | | | | |
| | SD | 54,540 | | | | | |
| | Positive number | 21 | | | | | |
| | Positive (%) | 21.9% | | | | | |
| | P (GC vs HD) | <0.001 | | | | | |
| CC | Average | 69,308 | | | | | |
| | SD | 58,449 | | | | | |
| | Positive number | 53 | | | | | |
| | Positive (%) | 27.6% | | | | | |
| | P (CC vs HD) | **<0.001** | | | | | |
| LC | Average | 62,148 | | | | | |
| | SD | 57,101 | | | | | |
| | Positive number | 24 | | | | | |
| | Positive (%) | 25.0% | | | | | |
| | P (LC vs HD) | **<0.001** | | | | | |
| MC | Average | 50,270 | | | | | |
| | SD | 36,802 | | | | | |
| | Positive number | 18 | | | | | |
| | Positive (%) | 18.8% | | | | | |
| | P (MC vs HD) | 0.0018 | | | | | |

### [Example 6]

### Comparison of serum antibody level to antigens; i.e., between KIAA0513 isoforms c and a

The serum antibody levels with respect to KIAA0513 isoform c (SEQ ID NO: 1) and KIAA0513 isoform a (SEQ ID NO: 2) were determined in healthy donors (HDs), acute ischemic stroke (AIS) patients, and cardiovascular disorder (CVD) patients through AlphaLISA.

As a result, with respect to both KIAA0513 isoforms c and a, the serum antibody levels were found to be higher in AIS patients and CVD patients than in HDs (see Figs. 7a and b). The reactivity of KIAA0513 isoform c to the serum antibody is higher than that of KIAA0513 isoform a. However, the relative reactivity values closely correlated with each other (Fig. 7c).

The test results have revealed that the main epitope site of a serum antibody to protein KIAA0513 is present in 301 amino acids of KIAA0513 isoform c.

### [Example 7]

### Correlation analysis between serum antibody level and test subject data

### (1) Mann-Whitney U analysis

Correlation analysis between s-KIAA0513-Ab levels and test subject data was carried out by use of 665 serum samples collected from subjects in Chiba Prefectural Sawara Hospital. The samples included 139 HD samples, 225 samples of AIS patients, 44 samples of TIA patients, 17 samples of asymptomatic ischemic cerebral stroke patients, 122 samples of deep and subcortical white matter hyperintensity (DSWMH) patients, 59 samples of chronic ischemic stroke (CIS) patients, and 41 control samples.

In the correlation analysis, Mann-Whitney U analysis was employed to compare s-KIAA0513-Ab levels with the parameters: sex (male/female), DM, hypertension (HT), CVD, lipidemia, and obesity ["normal or thin" when body mass index (BMI) is <25; and "obese" when BMI is ≥25], smoking habit, and drinking habit. As a result, a significant difference was found only in the case of presence/absence of HT (Table 6) .

**[Table 6]**

| Sex | | Male | Female |
|---|---|---|---|
| Sample number | | 396 | 268 |
| KIAA0513-Ab level | Average | 10,136 | 104,138 |
| | SD | 39,889 | 40,969 |
| P value (vs Male) | | | 0.1466 |

| Other disease | | DM- | DM+ |
|---|---|---|---|
| Sample number | | 525 | 135 |
| KIAA0513-Ab level | Average | 101,818 | 101,727 |
| | SD | 40,022 | 41,930 |
| P value (vs DM-) | | | 0.5031 |

| Other disease | | HT- | HT+ |
|---|---|---|---|
| Sample number | | 239 | 421 |
| KIAA0513-Ab level | Average | 96,476 | 104,821 |
| | SD | 38,988 | 40,905 |
| P value (vs HT-) | | | **0.0091** |

| Other disease | | CVD- | CVD+ |
|---|---|---|---|
| Sample number | | 623 | 37 |
| KIAA0513-Ab level | Average | 101,376 | 108,939 |
| | SD | 39,757 | 49,697 |
| *P* value (vs CVD-) | | | 0.1177 |

| Other disease | | Lipidemia- | Lipidemia+ |
|---|---|---|---|
| Sample number | | 475 | 185 |
| KIAA0513-Ab level | Average | 103,028 | 98,647 |
| | SD | 40,591 | 39,808 |
| P value (vs Lipidemia-) | | | 0.2716 |

| Obesity | | BMI < 25 | BMI ≥ 25 |
|---|---|---|---|
| Sample number | | 498 | 158 |
| KIAA0513-Ab level | Average | 102,071 | 101,390 |
| | SD | 40,475 | 40,488 |
| P value (vs Alcohol-) | | | 0.7490 |

| Life style | | Non-smoker | Smoker |
|---|---|---|---|
| Sample number | | 344 | 319 |
| KIAA0513-Ab level | Average | 98,991 | 104,832 |
| | SD | 38,563 | 42,055 |
| *P* value (vs non-smoker) | | | 0.0673 |

| Life style | | Alcohol- | Alcohol+ |
|---|---|---|---|
| Sample number | | 238 | 419 |
| KIAA0513-Ab level | Average | 102,663 | 101,364 |
| | SD | 39,883 | 40,759 |
| P value (vs Alcohol-) | | | 0.4595 |

### (2) Spearman's correlation analysis

Through Spearman's correlation analysis, correlations of s-KIAA0513-Ab level with continuous variables, specifically, general information parameters of subjects including age, height, weight, and BMI; degree of arteriostenosis including maximum intima-media thickening (max-IMT); life-style factors such as period of smoking (years) and alcohol frequency (/week); and blood test data, were investigated. Significant correlations were identified between s-KIAA0513-Abs level and age, max-IMT, ALP, potassium, C-reactive protein (CRP), body sugar (BS), and smoking period (see Table 7). Inverse correlations were observed between the antibody level and height and weight. Correlation between the antibody level and max-IMT indicates that the s-KIAA0513-Ab level is associated with stenosis and atherosclerosis.

Similar results were obtained using other cohorts. In Spearman's correlation analysis of a CKD cohort (300 cases), significant correlations of the antibody level were observed with plaque score, max-IMT, and CAVI (right), which are indices for atherosclerosis (see Table 8). In the CKD cohort, correlation was observed between CRP and the s-KIAA0513-Abs level, indicating certain association of inflammation. Meanwhile, in the CKD cohort, no significant correlations of the s-KIAA0513-Abs level were observed with age, height, weight, BMI, and potassium. AIS is closely related with age, and positive correlation was observed between the s-KIAA0513-Abs level and AIS. Therefore, the s-KIAA0513-Abs may be indirectly associated with aging.

**[Table 7]**

| Parameter | Number of XY pairs | | KIAA0513-Ab | |
|---|---|---|---|---|
| | | | *r* value | *P* value |
| Age | 663 | | 0.1959 | < **0.0001** |
| Height | 657 | | -0.1317 | **0.0007** |
| Weight | 661 | | -0.1093 | **0.0049** |
| BMI | 656 | | -0.0414 | 0.2899 |
| max-IMT | 457 | | 0.1763 | **0.0002** |
| AIG | 628 | | -0.0187 | 0.6402 |
| AST (GOT) | 655 | | 0.0395 | 0.3134 |
| ALT (GPT) | 655 | | -0.0035 | 0.9278 |
| ALP | 600 | | 0.0889 | **0.0295** |
| LDH | 631 | | 0.0431 | 0.2798 |
| tBil | 637 | | 0.0317 | 0.4250 |
| CHE | 506 | | -0.0387 | 0.3851 |
| γ-GTP | 609 | | -0.0099 | 0.8072 |
| TP | 633 | | -0.0184 | 0.6435 |
| ALB | 638 | | -0.0207 | 0.6014 |
| BUN | 654 | | -0.0454 | 0.2469 |
| CRE | 649 | | -0.0310 | 0.4303 |
| eGFR | 552 | | 0.0432 | 0.3113 |
| UA | 492 | | -0.0138 | 0.7596 |
| AMY | 415 | | -0.0901 | 0.0667 |
| T-CHO | 563 | | -0.0446 | 0.2913 |
| HDL-C | 435 | | -0.0280 | 0.5600 |
| TG | 460 | | -0.0198 | 0.6719 |
| Na | 641 | | -0.0347 | 0.3808 |
| K | 641 | | -0.0899 | **0.0228** |
| Cl | 641 | | -0.0392 | 0.3215 |
| Ca | 380 | | -0.0607 | 0.2376 |
| IP | 302 | | -0.0085 | 0.8824 |
| Fe | 311 | | -0.0083 | 0.8838 |
| CRP | 477 | | 0.0917 | **0.0453** |
| LDL-C | 344 | | -0.0559 | 0.3011 |
| WBC | 650 | | 0.0712 | 0.0697 |
| RBC | 650 | | -0.0220 | 0.5748 |
| HGB | | 650 | 0.0117 | 0.7651 |
| HCT | | 650 | -0.0041 | 0.9174 |
| MCV | | 650 | 0.0525 | 0.1814 |
| MCH | | 650 | 0.0611 | 0.1199 |
| MCHC | | 650 | 0.0401 | 0.3080 |
| RDW | | 650 | 0.0437 | 0.2661 |
| PLT | | 650 | -0.0269 | 0.4942 |
| MPV | | 650 | -0.0354 | 0.3683 |
| PCT | | 650 | -0.0271 | 0.4900 |
| PDW | | 650 | -0.0342 | 0.3838 |
| BS | | 596 | 0.0932 | **0.0229** |
| HbA1c | | 505 | -0.0212 | 0.6351 |
| Smoking period | | 656 | 0.1297 | **0.0009** |
| Alcohol frequency | | 662 | -0.0328 | 0.4000 |

| | | | | |
|---|---|---|---|---|
| *The data of the test subjects employed include the following: age, height, weight, body mass index (BMI), maximum intima-media thickness (max IMT), albumin/globulin ratio (A/G), aspartate aminotransferase (AST), alanine aminotransferase (ALT), alkaline phosphatase (ALP), lactate dehydrogenase (LDH), total bilirubin (tBil), cholinesterase (CHE), γ-glutamyl transpeptidase (y-GTP), total protein (TP), albumin (ALB), blood urea nitrogen (BUN), creatinine (CRE), estimated glomerular filtrating ratio (eGFR), uric acid (UA), amylase (AMY), total cholesterol (T-CHO), high-density lipoprotein cholesterol (HDL-C), triglyceride (TG), sodium (Na), potassium (K), chlorine (CI), calcium (Ca), inorganic phosphate (IP), iron (Fe), C-reactive protein (CRP), low-density lipoprotein cholesterol (LDL-C), white blood cell (WBC), red blood cell (RBC), hemoglobin (HGB), hamatocrit (HCT), mean corpuscular volume (MCV), mean corpuscular hemoglobin (MCH), MCH concentration (MCHC), red cell distribution width (RDW), platelet (PLT), mean platelet volume (MPV), procalcitonin (PCT), platelet distribution width (PDW), blood sugar (BS), and glycated hemoglobin (HbAlc), smoking period, alcohol frequency. **Table 7 shows the number of data, correlation coefficient (r), and P value in Spearman's correlation analysis. Values with significant correlation (P<0.05) are printed in boldface. | | | | |

**[Table 8]**

| | *r* value | *P* value |
|---|---|---|
| Age | 0.0640 | 0.2695 |
| Height | 0.0690 | 0.2342 |
| Weight | -0.0180 | 0.7567 |
| BMI | -0.0736 | 0.2045 |
| Plaque score | 0.2306 | < **0.0001** |
| max-IMT | 0.1157 | **0.0468** |
| CAVI (right) | 0.1244 | **0.0371** |
| CAVI (left) | 0.1133 | 0.0568 |
| ABI (right) | -0.0164 | 0.7804 |
| ABI (left) | -0.0559 | 0.3389 |
| HbA1c | -0.0890 | 0.2821 |
| W-PTH | 0.0502 | 0.3864 |
| Dia-period | -0.0176 | 0.7614 |
| ARB | -0.0294 | 0.6116 |
| ACE | 0.0977 | 0.0910 |
| PTA | 0.1276 | **0.0273** |
| Fe | -0.0456 | 0.4319 |
| Ferritin | 0.1655 | **0.0040** |
| TSAT Ratio | 0.0399 | 0.4910 |
| Kt/V | -0.0746 | 0.1975 |
| RBC | -0.0801 | 0.1664 |
| HGB | -0.0518 | 0.3713 |
| HCT | -0.0322 | 0.5787 |
| PLT | -0.1867 | **0.0012** |
| TP | -0.0368 | 0.5259 |
| ALB | -0.1337 | **0.0205** |
| UN | -0.1303 | **0.0240** |
| CRE | -0.0645 | 0.2655 |
| UA | -0.0570 | 0.3256 |
| Na | 0.0830 | 0.1515 |
| K | -0.0043 | 0.9405 |
| CI | 0.0494 | 0.3936 |
| Ca | 0.0039 | 0.9459 |
| IP | -0.0242 | 0.6768 |
| Mg | 0.0675 | 0.2436 |
| AST | 0.0816 | 0.1586 |
| ALT | 0.0111 | 0.8486 |
| LDH | 0.0591 | 0.3076 |
| γ-GTP | 0.0811 | 0.1610 |
| ALP | 0.0031 | 0.9574 |
| tBil | 0.0344 | 0.5530 |
| AMY | -0.0151 | 0.7940 |
| CK | -0.0239 | 0.6799 |
| T-CHO | -0.0655 | 0.2583 |
| HDL-C | -0.1534 | **0.0078** |
| LDL-C | -0.0099 | 0.8640 |
| TG | 0.0292 | 0.6147 |
| CRP | 0.1813 | **0.0016** |

| | | |
|---|---|---|
| Mode of representation of values printed in boldface, and means of abbreviation are the same as those employed in Table 7. | | |

### [Summary of the Examples]

Through primary screening by use of a human protein microarray, protein KIAA0513 was identified as an antigen recognized by a serum antibody of atherosclerosis patients. Then, a recombinant GST-tagged protein KIAA0513 was purified. By use of the thus-purified protein as an antigen, the serum antibody level was determined through AlphaLISA. As a result, the s-KIAA0513-Abs level was found to be significantly higher in patients of AIS, TIA, DM, CVD, OSA, CKD, EC, GC, CC, LC, and MC than in HDs (see Figs. 1 to 5, Tables 1 to 5). Among these diseases, a particularly high AUC value was observed in DM, Type 2 CKD, and EC (see Figs. 2, 4, and 5). Furthermore, through correlation analysis, the serum antibody level to protein KIAA0513 was found to positively correlate with max-IMT, plaque score, and CAVI, which are indices for atherosclerosis-related lesions (see Tables 7 and 8). In contrast, the serum antibody level to protein KIAA0513 was found to weakly correlate with BS (P=0.0229) (see Table 7), but no correlation was found between the serum antibody level and HbAlc, which is a typical DM marker (see Table 7). The serum antibody level to protein KIAA0513 was relatively higher in DM patients than in HDs. However, conceivably, the antibody marker does not mainly reflect an elevated blood sugar level in DM, but shows an arteriosclerosis lesion induced by DM. Also, angiogenesis is an essential element for the progress of solid cancers such as EC. Thus, similar to arteriosclerosis, dysplasia of blood vessels in a solid cancer is thought to promote formation of protein KIAA0513. Actually, DM and arteriosclerosis are known to be risk factors for cancer.

Currently, no precise function of protein KIAA0513 has been elucidated. However, protein KIAA0513 has been reported to bind to a protein involved in synaptic transmission and apoptosis induction. Thus, conceivably, protein KIAA0513 may be involved in at least such signal transmission.

Generally, humoral antibody markers exhibit a higher sensitivity of detecting data concerning diseases, as compared with humoral antigen markers. Among humoral antigens, a KIAA0513 antigen has particularly high antigenicity. Thus, the anti-KIAA0513 humoral antibody marker exhibits remarkably high sensitivity. The above experiments have revealed that the present invention can acquire data concerning the potential or actual presence of diseases (e.g., cancers, cerebral infarction, myocardial infarction, diabetes mellitus, and chronic kidney disease) which are fatal to humans, as well as data showing the presence of an artery lesion (e.g., arteriosclerosis, or anomalous angiogenesis in solid cancer).

### Industrial Applicability

Hitherto, there have been known only a small number of easily employable markers for life-threatening grave diseases including cancers, cerebral infarction, myocardial infarction, diabetes mellitus, chronic kidney disease, etc., and individual disease-specific markers must be used. However, the data acquisition method of the present invention enables to acquire data concerning diseases at remarkably high sensitivity and to acquire comprehensive data concerning grave diseases. In addition, by acquiring data showing the presence of an artery lesion (e.g., arteriosclerosis, or anomalous angiogenesis in solid cancer), it is possible to use the acquired data as an index for disease prevention. Thus, the method of the present invention is suited for mass examination or the like.

As described above, according to the present invention, the presence of pre-existing conditions, which are factors for aggravation of an infection, can be comprehensively detected by use of a single biomarker. If the data acquisition method of the present invention is carried out in mass examination in response to epidemic of respiratory tract infection caused by novel coronavirus (COVID-19) or an influenza virus, hospital capacity including medical staff, beds, etc. can be effectively furnished, and better treatment for the infection can be attained.

## Claims

1. A method for acquiring data, the method comprising: determining a level of an antibody to a protein having an amino acid sequence represented by SEQ ID NO: 1 or 2 which forms protein KIAA0513, or a portion of the protein, or to a protein having an amino acid sequence represented by SEQ ID NO: 1 or 2 in which 10% or less of the amino acid residues (the decimal point being suppressed) are deleted, substituted, or added, or a portion of the protein, the antibody being potentially present in a body fluid sample collected from a living body; and acquiring data showing a rise in the antibody level as data showing the presence of an artery lesion.

2. A method for acquiring data, the method comprising: determining a level of an antibody to a protein having an amino acid sequence represented by SEQ ID NO: 1 or 2 which forms protein KIAA0513, or a portion of the protein, or to a protein having an amino acid sequence represented by SEQ ID NO: 1 or 2 in which 10% or less of the amino acid residues (the decimal point being suppressed) are deleted, substituted, or added, or a portion of the protein, the antibody being potentially present in a body fluid sample collected from a living body; and acquiring data showing a rise in the antibody level as data showing the potential or actual presence of cerebrovascular or cardiovascular disorder, diabetes mellitus, chronic kidney disease, or a solid cancer.

3. The data acquisition method according to claim 2, wherein the data showing the potential or actual presence of cerebrovascular or cardiovascular disorder, diabetes mellitus, chronic kidney disease, or a solid cancer are acquired as data concerning an artery lesion involved in the diseases.

4. The data acquisition method according to any one of claims 1 to 3, wherein a portion of the protein having an amino acid sequence represented by SEQ ID NO: 1 has 10 to 301 amino acid residues.

5. The data acquisition method according to any one of claims 1 to 4, wherein the body fluid sample collected from a living body is a blood sample.

6. The data acquisition method according to claim 5, wherein the blood sample is a serum or plasma sample.

7. The data acquisition method according to any one of claims 1 to 6, wherein the antibody level is determined through immuno-chromatography, ELISA, AlphaLISA, indirect immunofluorescence, Western blotting (immunoblotting), turbidimetry, nephelometry, latex-coagulation turbidimetry, or CLEIA.

8. A kit for acquiring data, the kit being employed in carrying out the data acquisition method as recited in any one of claims 1 to 7.
